(19) Europäisches Patentamt
European Patent Office
Office européen des brevets

(11) **EP 4 550 943 A1**

(12) **EUROPEAN PATENT APPLICATION**
published in accordance with Art. 153(4) EPC

(43) Date of publication:
**07.05.2025 Bulletin 2025/19**

(21) Application number: **23830694.8**

(22) Date of filing: **11.01.2023**

(51) International Patent Classification (IPC):
**H05H 13/02** (2006.01)    **A61N 5/10** (2006.01)

(52) Cooperative Patent Classification (CPC):
**A61N 5/10; H05H 13/02**

(86) International application number:
**PCT/JP2023/000454**

(87) International publication number:
**WO 2024/004238 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.07.2022   JP 2022106868**

(71) Applicant: **HITACHI HIGH-TECH CORPORATION Tokyo 105-6409 (JP)**

(72) Inventor: **NAKASHIMA, Yuto Tokyo 100-8280 (JP)**

(74) Representative: **MERH-IP Matias Erny Reichl Hoffmann Patentanwälte PartG mbB Paul-Heyse-Straße 29 80336 München (DE)**

(54) **ACCELERATOR AND PARTICLE BEAM THERAPY DEVICE**

(57)    A circular accelerator (30) is a device that applies a static magnetic field that circulates a charged particle beam, a radio frequency acceleration electric field that is frequency-modulated and accelerates the charged particle beam, and a radio frequency disturbance electric field that extracts the charged particle beam, in which a circulation frequency of the charged particle beam circulating inside the circular accelerator (30) or kinetic energy of the charged particle beam is obtained after stopping the radio frequency acceleration electric field, and the radio frequency disturbance electric field is generated according to the obtained circulation frequency or kinetic energy. As a result, an accelerator and a particle therapy apparatus capable of improving beam utilization efficiency as compared with the related art are provided.

*FIG. 2*

EP 4 550 943 A1

## Description

Technical Field

The present invention relates to an accelerator and a particle therapy apparatus.

Background Art

PTL 1 describes, as an example of a synchrocyclotron for extracting beams of various energies, that the synchrocyclotron includes a magnetic unit including a valley sector and a hill sector, is configured to create a z component of a main magnetic field characterized by a radial tune of successive orbits other than 1 and included within 1±0.1 for all values of an average radius included between a low radius and a high radius corresponding to respective average radial positions of charged particles at low and high energies.

PTL 2 describes that in a circular accelerator that accelerates a charged particle beam while increasing an orbital radius by applying a radio frequency in a main magnetic field, the charged particle beam is extracted by applying a radio frequency different from the radio frequency used for acceleration to the charged particle beam.

NPL 1 describes an example of a compact superconducting synchrocyclotron as a part of a small footprint proton beam therapy system.

Citation List

Patent Literature

      PTL 1: US 11,160,159
      PTL 2: JP 2019-133745 A

Non-Patent Literature

NPL 1: W. Kleeven, "The IBA Superconducting Synchrocyclotron Project S2C2", Proceedings of Cyclotrons 2013

Summary of Invention

Technical Problem

In particle therapy, which is one of cancer therapies, a target volume is irradiated with a charged particle beam of protons, carbon ions, or the like. In a particle therapy apparatus used for particle therapy, energy and spatial spread of a charged particle beam are adjusted to form dose distribution matching a shape of the target volume. The particle therapy apparatus includes an accelerator, a beam transport system, and an irradiation device.

The accelerator is a device that accelerates a charged particle beam to energy used for therapy, and examples of the accelerator used for particle therapy include a synchrotron, a cyclotron, and a synchrocyclotron.

Downsizing of the particle therapy apparatus is achieved by downsizing of the accelerator. The accelerator can be downsized by using a superconducting magnet as a magnet for bending the charged particle beam.

Examples of the accelerator to which the superconducting magnet is applied include a synchrocyclotron described in NPL 1. In the synchrocyclotron, a particle beam circulates in a static magnetic field formed by a superconducting coil, and is accelerated by a radio frequency acceleration electric field synchronized with the circulation of the particle beam. In the synchrocyclotron, since a circulation frequency of the beam decreases with the acceleration, a frequency of the radio frequency acceleration electric field is modulated according to the circulation frequency. A horizontal plane orbit of the beam in the synchrocyclotron becomes concentric for each energy, and the beam reaching the design maximum energy is extracted from an extraction channel. In order to use the extracted beam for therapy, it is necessary to decelerate with a scatterer so as to have energy corresponding to a depth of the target volume.

On the other hand, there are circular accelerators described in PTL 1 and PTL 2 as accelerators in which energy of an extracted beam is variable.

The circular accelerator described in PTL 1 extracts beams of various energies that circulate concentrically by exciting a dynamic magnetic field that disturbs a beam with a coil in a conventional synchrocyclotron.

In the circular accelerator described in PTL 2, main magnetic field distribution is formed such that circulation orbits of beams having different energies are eccentric from the center of the circular accelerator to one side in a radial direction, and a beam having specific energy is extracted by increasing a betatron oscillation amplitude by a radio frequency electric field generated in a region where beam orbits are aggregated and passing a disturbing magnetic field through the region.

There is a scanning irradiation method as a method of forming an extracted beam in dose distribution matching the shape of the target volume. In the scanning irradiation method, the dose distribution is formed by scanning the beam using a scanning magnet installed upstream of the target volume.

In the case of increasing the betatron oscillation amplitude of the beam by using the radio frequency electric field in the circular accelerator, it is necessary to apply the radio frequency electric field having a frequency determined from the circulation frequency of the beam and a betatron oscillation frequency of the beam. However, it has become clear that, in a case where there is a deviation in frequency, an increase in betatron oscillation amplitude is suppressed, which may lead to a decrease in the final beam utilization efficiency, and it has become clear that improvement is desired.

The present invention provides an accelerator and a particle therapy apparatus capable of improving beam utilization efficiency as compared with the related art.

Solution to Problem

The present invention includes a plurality of means for solving the above-described problems, and examples thereof include a circular accelerator that applies a static magnetic field that circulates a charged particle beam, a radio frequency acceleration electric field that is frequency-modulated and accelerates the charged particle beam, and a radio frequency disturbance electric field that extracts the charged particle beam, in which a circulation frequency of the charged particle beam circulating inside the circular accelerator or kinetic energy of the charged particle beam is obtained after stopping the radio frequency acceleration electric field, and the radio frequency disturbance electric field is generated according to the obtained circulation frequency or kinetic energy.

Advantageous Effects of Invention

According to the present invention, beam utilization efficiency can be improved as compared with the related art. Problems, configurations, and effects other than those described above will become apparent by the following description of embodiments.

Brief Description of Drawings

[FIG. 1] FIG. 1 is an external view of a circular accelerator according to a first embodiment.
[FIG. 2] FIG. 2 is a cross-sectional view of the circular accelerator according to the first embodiment.
[FIG. 3] FIG. 3 is a view taken along arrow A-A' of FIG. 2.
[FIG. 4] FIG. 4 is a view taken along arrow B-B' of FIG. 2.
[FIG. 5] FIG. 5 is a control flowchart of the circular accelerator according to the first embodiment.
[FIG. 6] FIG. 6 is a control chart of the circular accelerator according to the first embodiment.
[FIG. 7] FIG. 7 illustrates table data 1 of the circular accelerator according to the first embodiment.
[FIG. 8] FIG. 8 illustrates table data 2 of the circular accelerator according to the first embodiment.
[FIG. 9] FIG. 9 illustrates table data 3 of the circular accelerator according to the first embodiment.
[FIG. 10] FIG. 10 is an external view of a circular accelerator according to a second embodiment.

[FIG. 11] FIG. 11 is a cross-sectional view of the circular accelerator according to the second embodiment.
[FIG. 12] FIG. 12 is a view taken along arrow C-C' of FIG. 11.
[FIG. 13] FIG. 13 is a control chart of the circular accelerator according to the second embodiment.
[FIG. 14] FIG. 14 illustrates table data 1 of the circular accelerator according to the second embodiment.
[FIG. 15] FIG. 15 illustrates table data 2 of the circular accelerator according to the second embodiment.
[FIG. 16] FIG. 16 is a control flowchart of the circular accelerator according to the second embodiment.
[FIG. 17] FIG. 17 is an overall configuration diagram of a particle therapy apparatus according to a third embodiment.

Description of Embodiments

Hereinafter, embodiments of an accelerator and a particle therapy apparatus according to the present invention will be described with reference to the drawings. Note that, in the drawings used in the present specification, the same or corresponding constituent elements are denoted by the same or similar reference signs, and a repeated description of the constituent elements may be omitted.

<First Embodiment>

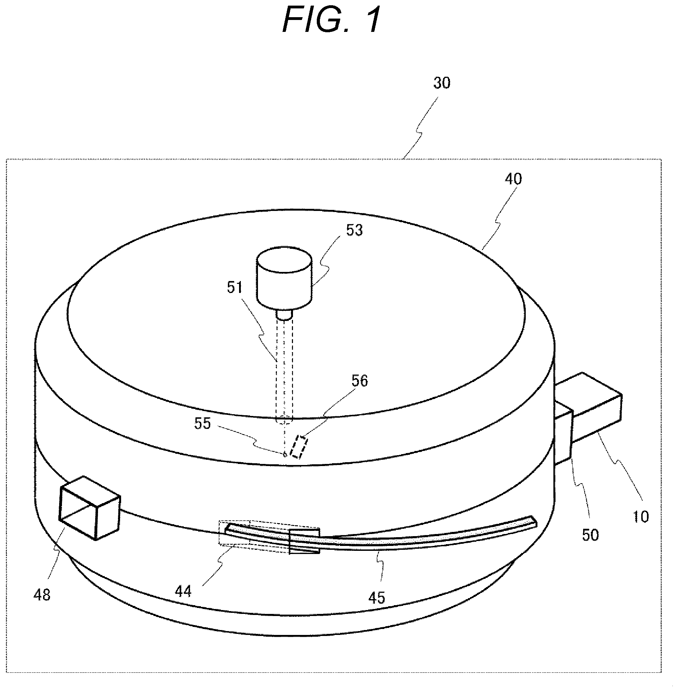

A first embodiment of an accelerator according to the present invention will be described with reference to FIGS. 1 to 9.

First, an overall configuration of a circular accelerator 30 will be described with reference to FIG. 1. FIG. 1 illustrates an external view of the circular accelerator 30.

The circular accelerator 30 according to the present embodiment illustrated in FIG. 1 applies a static magnetic field that circulates a charged particle beam, a radio frequency acceleration electric field that is frequency-modulated and accelerates the charged particle beam, and a radio frequency disturbance electric field that extracts the charged particle beam, an outer shell thereof is formed by a main magnet 40 that can be divided in a vertical direction, and a beam acceleration region inside the main magnet 40 is kept vacuum.

An ion source 53 that generates a beam of ions to be injected to the main magnet 40 is installed above the main magnet 40. The beam generated by the ion source 53 passes through a low energy beam transport system 51 and is injected to the beam acceleration region where the beam is accelerated inside the main magnet 40 from an ion injection portion 55 provided near the center of a main magnetic pole 35.

As the ion source 53, an ECR ion source, a laser ion source, or the like can be applied. In a case where ions are injected from the outside, the ions are injected to the beam acceleration region through an electrostatic inflector 56, for example. The ion source 53 may be disposed inside the vacuumed beam acceleration region inside the main magnet 40, and in this case, a PIG type ion source or the like is suitable.

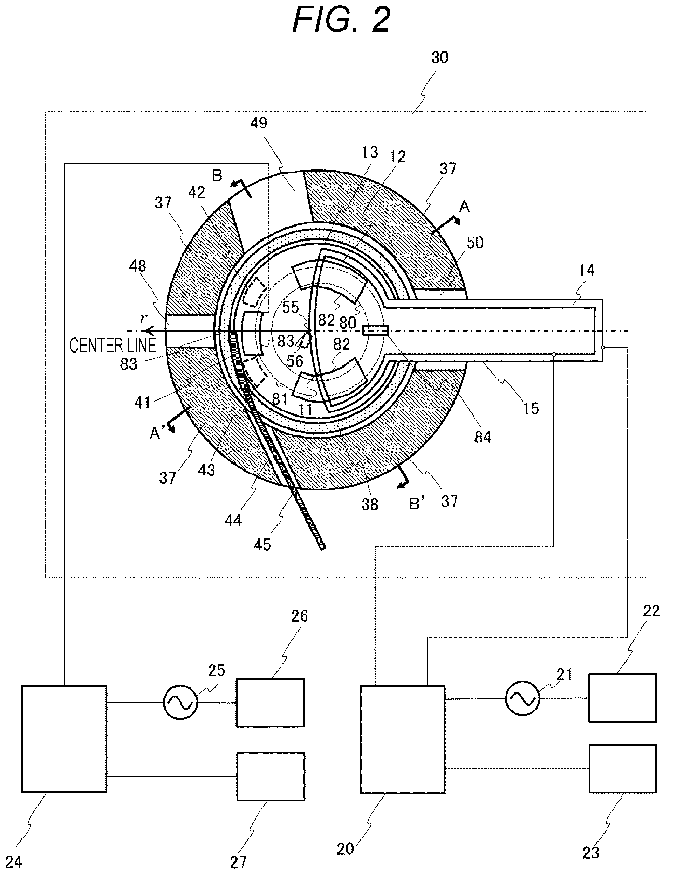

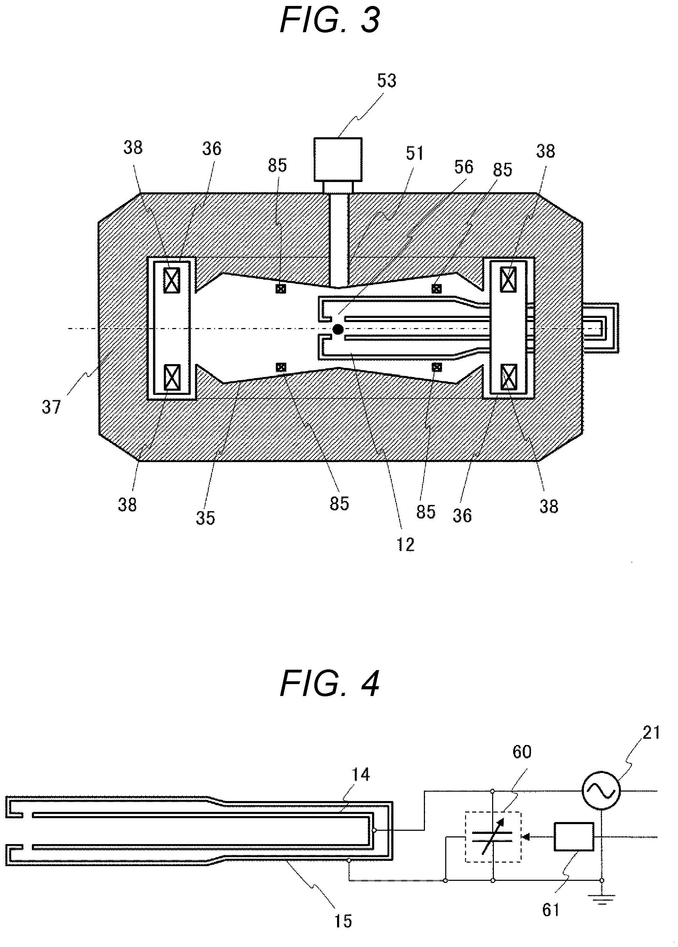

FIG. 2 is a cross-sectional view taken along the center plane of the circular accelerator 30, and FIG. 3 is a vertical cross-sectional view of the circular accelerator 30 (a view taken along arrow A-A' of FIG. 2).

The main magnet 40 includes the main magnetic pole 35, a yoke 37, and a main coil 38. The yoke 37 forms an appearance of the main magnet 40 and has a substantially cylindrical region therein. The main coil 38 is an annular coil and is installed along an inner wall of the yoke 37. The main coil 38 is a superconducting coil, and a cryostat 36 is installed around the main coil 38 for cooling.

The main magnetic pole 35 is disposed in an opposing vertical arrangement on an inner circumferential side of the main coil 38. A magnetic field formed by the energized main coil 38 and the 42 main magnetic pole 35 is referred to as a main magnetic field. The acceleration region is a region for accelerating the beam in the main magnetic field.

The yoke 37 has a plurality of through holes. A beam through hole 44 for extracting an accelerated beam, a coil through hole 48 for drawing various coil conductors inside the yoke 37 to the outside, a vacuum evacuation through hole 49, and a radio frequency system through hole 50 for a radio frequency acceleration cavity 10 are provided in a connection surface of the yoke 37.

The radio frequency acceleration cavity 10 is a resonance-type cavity, and includes a dee electrode 12, a dummy dee electrode 13, an inner conductor 14, and an outer conductor 15.

The dee electrode 12 is a D-shaped hollow electrode and is connected to the inner conductor 14. The dummy dee electrode 13 is an electrode connected to the outer conductor 15 that externally covers the inner conductor 14, and has a ground potential. The dummy dee electrode 13 forms an acceleration gap 11 with the dee electrode 12. An acceleration voltage frequency-modulated by a radio frequency acceleration voltage control device 20 is generated in the acceleration gap 11 between the dee electrode 12 and the dummy dee electrode 13. The acceleration gap 11 illustrated in FIG. 2 indicates a case where the number of harmonics is 1, that is, a case where the circulation frequency and an acceleration frequency are the same. A shape of the acceleration gap 11 is formed according to an orbit shape of the beam.

Here, a beam behavior from the injection to the extraction in the circular accelerator 30 will be described.

The beam injected from the ion source 53 is accelerated by a radio frequency electric field and circulates in the main magnetic field while increasing energy. As the beam is accelerated, a radius of curvature of the orbit increases to form a spiral orbit.

Here, in the beam acceleration region, an orbit through which the beam starts to be accelerated and reaches the maximum energy is referred to as a closed orbit. Among the closed orbits, an orbit through which the beam having the minimum energy passes is referred to as a minimum energy orbit 80, an orbit through which the beam having the maximum energy passes is referred to as a maximum energy orbit 81, and a plane in which the closed orbit draws a spiral is referred to as an orbital plane. An axis in a radial outward direction from the center in a two-dimensional polar coordinate system of the orbital plane with the center of the acceleration region as the origin is defined as an r axis. The main magnetic field satisfies a beam stabilization condition that an n value represented by the following Formula (1) is larger than 0 and smaller than 1.

[Math. 1]

$$n = -\frac{\rho}{|B_z|}\frac{\partial B_z}{\partial r} \quad \cdots (1)$$

In Formula (1), $\rho$ represents a bending radius of a design orbit, $B_z$ represents a magnetic field intensity, and $\partial B_z/\partial r$ represents a magnetic field gradient in an r direction. At this time, a beam slightly deviated in the radial direction from the design orbit receives a restoring force to return to the design orbit, and at the same time, a beam deviated in a direction perpendicular to the orbit plane also receives a restoring force from the main magnetic field in a direction to return to the orbit plane. Such oscillation is referred to as betatron oscillation, and the frequency of the oscillation is referred to as a betatron oscillation frequency.

$\partial B_z/\partial r$ of the main magnetic field is designed such that the betatron oscillation of the beam is made in the vicinity of the design orbit and the beam can stably circulate and be accelerated. In addition, a frequency per turn is referred to as a tune, and displacement of the beam on the r axis to the outside of the orbit plane per turn is referred to as turn separation. The betatron oscillation of the circulating beam in a direction orthogonal to the orbit of the beam in the orbit plane is referred to as betatron oscillation in a horizontal direction, and the tune is referred to as a horizontal tune. An amplitude of the betatron oscillation increases due to resonance when an appropriate radio frequency voltage is applied. In addition, for the beam of all energies, a betatron oscillation frequency (horizontal tune) $\nu r$ in a direction parallel to the orbital plane and orthogonal to the orbit is set to a value close to 1.

The main magnetic field distribution described above is formed by the main magnetic pole 35 and a trim coil or magnetic pole piece installed on the surface of the main magnetic pole 35. Since constituent elements forming the main magnetic field distribution are arranged symmetrically with respect to the orbital plane, the main magnetic field has only a magnetic field component in a direction perpendicular to the orbital plane on the orbital plane. A radio frequency acceleration voltage frequency control device 23 includes a variable capacitor 60 and a variable capacitor control device 61 illustrated in FIG. 4.

In the present embodiment, a rotating capacitor is used as the variable capacitor 60. At this time, what is controlled by the variable capacitor control device 61 is rotation of a motor connected to the rotating capacitor or the like.

The circular accelerator 30 according to the present embodiment includes a kicker coil 85, a radio-frequency kicker 83, a septum coil 41, and a high energy beam transport system 45 as devices for extracting a beam.

The radio-frequency kicker 83 is a device for further applying a dynamic magnetic field that kicks the charged particle beam circulating in the circular accelerator 30 to a generation region of the radio frequency disturbance electric field by applying a radio frequency voltage to the circulating beam passing therethrough.

In addition, a peeler magnetic field region 42 and a regenerator magnetic field region 43, which are the radio frequency disturbance magnetic fields including a bipolar magnetic field and a multipolar magnetic field, are formed inside the main magnet 40.

The radio-frequency kicker 83, the peeler magnetic field region 42, the regenerator magnetic field region 43, the septum coil 41, and the high energy beam transport system 45 are used for beam extraction. By exciting the kicker coil 85 with respect to the circulating beam, the beam reaches a position of the radio-frequency kicker 83.

Thereafter, a voltage is supplied from a radio frequency extraction power supply 25 according to a command from a radio frequency extraction power supply control device 26, and a radio frequency electric field is generated by the radio-frequency kicker 83 through a radio frequency extraction voltage control device 24 that implements a frequency indicated by a radio frequency extraction voltage frequency control device 27. The radio frequency electric field increases the betatron oscillation amplitude of the beam. The beam with the increased betatron oscillation amplitude eventually reaches the peeler magnetic field region 42 and the regenerator magnetic field region 43 installed at a distance from the maximum energy orbit 81 on an outer circumferential side of the maximum energy orbit 81.

The beam that has reached the peeler magnetic field region 42 is kicked to an outer circumferential side of the orbit plane, whereas the beam that has reached the regenerator magnetic field region 43 is kicked to an inner circumferential side of the orbit plane. Kicking by a quadrupole magnetic field component of the peeler magnetic field region 42 further increases the betatron oscillation amplitude and increases the turn separation. At the same time, the magnetic field of the regenerator magnetic field region 43 prevents the horizontal tune of the beam from fluctuating abruptly, thereby preventing the beam from being lost due to divergence of the betatron oscillation in the vertical direction orthogonal to the horizontal direction by 90 degrees before the beam is extracted.

When sufficient turn separation is obtained, the beam enters the septum coil 41, is kicked outside the orbital plane, passes through the high energy beam transport system 45, and is extracted outside the circular accelerator 30.

An increase width of the turn separation by the peeler magnetic field region 42 and the regenerator magnetic field region 43 is much larger than that by the radio-frequency kicker 83. Therefore, it is possible to adjust the amount of beams reaching the peeler magnetic field region 42 and the regenerator magnetic field region 43 among the beams circulating on the maximum energy orbit 81 by adjusting the radio frequency voltage applied by the radio-frequency kicker 83.

As a result, when the application of the radio frequency to the radio-frequency kicker 83 is stopped during the beam extraction, the beam does not reach the peeler magnetic field region 42 and the regenerator magnetic field region 43, and the beam extraction from the circular accelerator 30 can be interrupted. Similarly, it is also possible to resume the extraction of the beam by resuming the application to the radio-frequency kicker 83.

In addition, an intensity of the beam extracted from the circular accelerator 30 can be controlled by controlling an intensity of the voltage applied to the radio-frequency kicker 83 and any one of an amplitude, phase, and frequency of the radio frequency.

The beam traveling inside the septum coil 41 is bent and transported to the high energy beam transport system 45. Two or more septum coils 41 may be disposed in a beam traveling direction. In a case where the beam can be transported by the magnetic field formed only by the main magnet 40, the septum coil 41 does not have to be provided.

The high energy beam transport system 45 for transporting the extracted beam from the inside to the outside of the main magnet 40 is disposed so as to extend from the septum coil 41 to the outside of the main magnet 40 through the beam through hole 44.

The peeler magnetic field region 42 and the regenerator magnetic field region 43 are regions where the multipolar magnetic field acting on the beam exists. The multipolar magnetic field includes at least a quadrupole magnetic field component, and may include a multipolar magnetic field of four or more poles, or a bipolar magnetic field. The peeler magnetic field region 42 has a magnetic field gradient in a direction in which the main magnetic field is weakened toward a radially outer circumferential side, and the regenerator magnetic field region 43 has a magnetic field gradient in a direction in which the main magnetic field is strengthened toward the radially outer circumferential side. As the peeler magnetic field region 42, a region where the main magnetic field at a magnetic pole end portion decreases can also be used.

The peeler magnetic field region 42 and the regenerator magnetic field region 43 are arranged in an azimuthal angle region sandwiching a beam extraction path entrance 82 on the outer circumferential side of the maximum energy orbit 81. In addition, it is desirable that the peeler magnetic field region 42 and the regenerator magnetic field region 43 are disposed on the outer circumferential side with a width larger than an amplitude of the betatron oscillation before resonance from the maximum energy orbit 81 such that the beam does not travel to the peeler magnetic field region 42 or the regenerator magnetic field region 43 before the betatron oscillation amplitude is increased by the radio-frequency kicker 83.

In addition, it is desirable to dispose the peeler magnetic field region 42 upstream and dispose the regenerator magnetic field region 43 downstream in the beam traveling direction, and vice versa.

In the vicinity of the peeler magnetic field region 42 and the regenerator magnetic field region 43, a plurality of magnetic pole pieces or a coil made of a magnetic material, or both are fixedly disposed by a non-magnetic material to form a desired multipolar magnetic field. For example, for each of the peeler magnetic field region 42 and the regenerator magnetic field region 43, the multipolar magnetic field is formed by a plurality of pole pieces, and the bipolar magnetic field is formed by a coil. The plurality of magnetic pole pieces and the coil can be disposed close to each other or can be disposed at spatially separated positions.

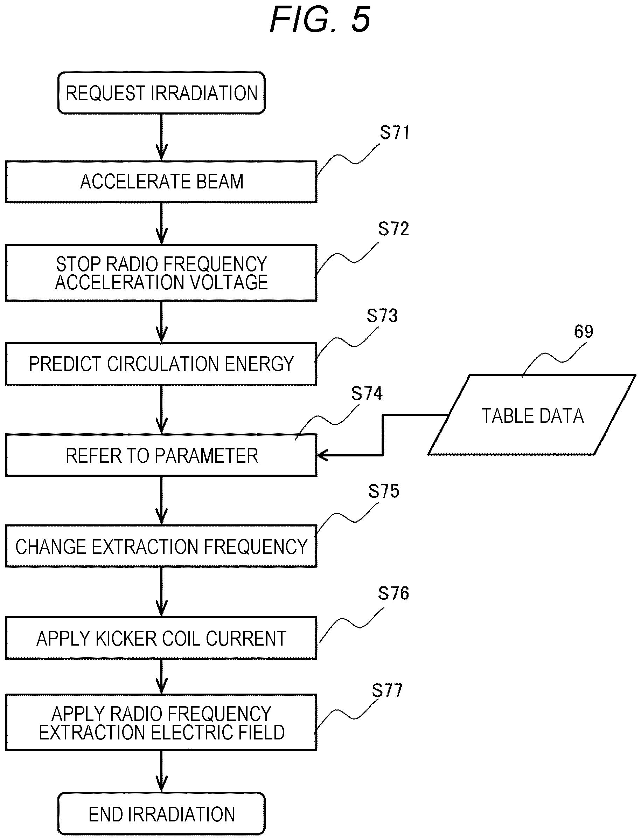

FIG. 5 illustrates a flowchart until irradiation to the outside of the accelerator is completed in a case where irradiation with a beam of certain energy is requested.

After the irradiation is requested, acceleration of a beam accelerated to required energy by beam acceleration (S71) is stopped by stopping voltage supply from a radio frequency acceleration power supply 21 (S72) by a radio frequency acceleration power supply control device 22, and the beam is accumulated in the accelerator.

In the present embodiment, after a radio frequency acceleration voltage stop 62, the radio frequency acceleration voltage control device 20 obtains the circulation frequency of the charged particle beam circulating inside the circular accelerator 30 or kinetic energy of the charged particle beam (S73), and the radio frequency extraction voltage control device 24 generates the radio frequency disturbance electric field according to the obtained circulation frequency or kinetic energy.

Here, in the present embodiment, as a specific example of a method of obtaining the circulation frequency of the charged particle beam circulating in the circular accelerator 30 or the kinetic energy of the charged particle beam, any one of the following methods can be adopted: (1) obtaining the circulation frequency or the kinetic energy by measuring the circulation frequency or the kinetic energy; (2) obtaining the circulation frequency or the kinetic energy based on a command value for the dee electrode 12 to which the radio frequency acceleration electric field is applied; (3) obtaining the circulation frequency or the kinetic energy based on a measured value of the radio frequency acceleration electric field; and (4) obtaining the circulation frequency or the kinetic energy based on a stop time of the radio frequency acceleration electric field.

In the method (1), for example, a circulation position or the circulation frequency of the charged particle beam circulating inside the circular accelerator 30 is measured by a measurement electrode 84 in FIG. 2, and the circulation frequency or the kinetic energy is obtained in the radio frequency acceleration voltage control device 20 from the measured circulation position or circulation frequency.

In the method (2), the circulation frequency or the kinetic energy is calculated from a command value of a radio frequency acceleration voltage output to the radio frequency acceleration power supply 21 immediately before the stop. For example, a relationship with the circulation frequency or the kinetic energy is obtained based on the minimum value and the maximum value of the command value, and the circulation frequency or the kinetic energy is obtained through interpolation.

In the method (3), for example, in the radio frequency acceleration voltage stop 62, the circulation frequency is obtained from the radio frequency acceleration voltage generated by the radio frequency acceleration power supply 21 immediately before the stop, and the kinetic energy is obtained from a rotation angle of the motor connected to the rotating capacitor. By expressing the radio frequency acceleration voltage or a rotation angle of the motor as a function with respect to an acceleration start time, the circulation frequency or the kinetic energy is obtained by performing back calculation as the circulation frequency or the kinetic energy of the circulating beam.

In the method (4), the circulation frequency or the kinetic energy is obtained from an output timing of a command of the radio frequency acceleration voltage stop 62.

Preferably, the extraction frequency is changed (S75) by referring to a parameter (the circulation frequency or the kinetic energy) obtained in S73 by any one of the methods (1) to (4) (S74), a kicker coil current for implementing the changed extraction frequency is applied (S76), and a radio frequency extraction electric field is applied (S77).

Here, the betatron oscillation has a property that the amplitude increases resonantly when a product of either the tune or a fractional part of the tune and the circulation frequency of the beam is substantially the same as the frequency of the applied radio-frequency voltage for extraction.

Therefore, a frequency $f_{ext}$ of the radio-frequency voltage for extraction is set to be substantially the same as a product $\Delta v_r \times f_{rev}$ of a fractional part $\Delta v_r$ of the horizontal tune $v_r$ of the maximum energy beam and a circulation frequency $f_{rev}$ of the maximum energy beam. As a result, the amplitude of the horizontal betatron oscillation continues to increase resonantly, and the beam eventually reaches the peeler magnetic field region 42 and the regenerator magnetic field region 43.

The frequency $f_{ext}$ of the frequency voltage may be equal to a product $v_r \times f_{rev}$ of the horizontal tune $v_r$ of the maximum energy beam and the circulation frequency $f_{rev}$ of the maximum energy beam.

The beam is kicked to the outer circumferential side when passing through the peeler magnetic field region 42, and is kicked to the inner circumferential side when passing through the regenerator magnetic field region 43. Since both the peeler magnetic field region 42 and the regenerator magnetic field region 43 have a magnetic field gradient in the radial direction, the amount of kicking gradually increases while the beam circulates a plurality of times, and the turn separation increases. That is, the turn separation can be increased by using a resonance condition of the betatron oscillation of $2v_r = 2$.

Eventually, when the turn separation greatly exceeding a thickness of a coil conductor installed on the inner circumferential side of the septum coil 41 is obtained, the beam is guided into the septum coil 41, is sufficiently bent, is guided to the high energy beam transport system 45, and is extracted.

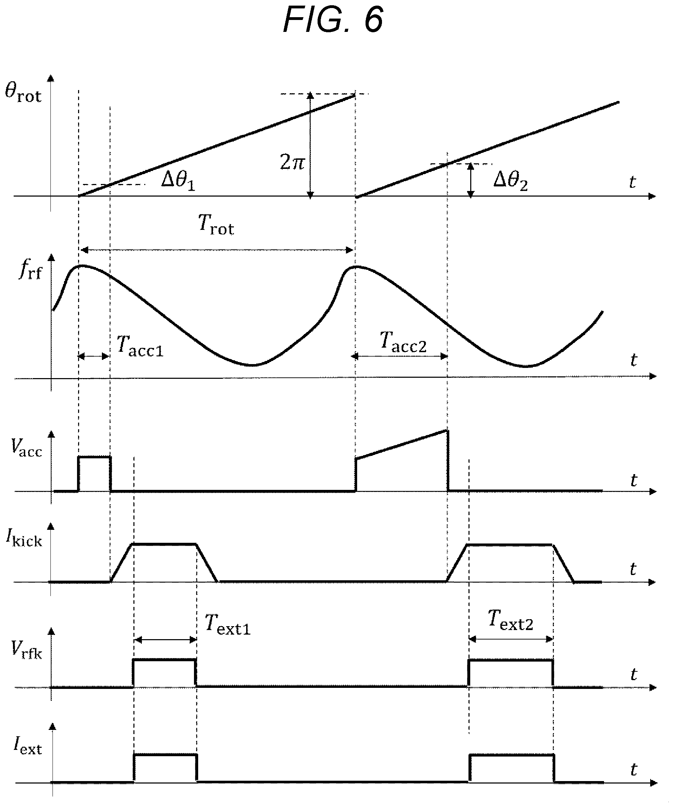

FIG. 6 illustrates a control chart according to the present embodiment. $\theta$rot is the rotation angle of the motor connected to the rotating capacitor. Here, a rotation speed is constant, but the rotation speed may be varied. $f_{rf}$ is a frequency of the radio frequency acceleration voltage, and is a frequency that is an integral multiple of a rotation frequency of the motor. $V_{acc}$ is an amplitude of the radio frequency acceleration voltage, and is applied and stopped according to required beam energy. At this time, intensity modulation may be performed to adjust a radio frequency bucket area. $I_{kicker}$ is a kicker coil current required to reach the radio-frequency kicker 83, and becomes constant after a certain rise time due to an inductance of the coil.

At the same time, by applying a radio-frequency voltage for extraction $V_{rfk}$, the betatron oscillation amplitude in the horizontal direction increases, and the beam is finally extracted to the outside of the accelerator and measured as an extraction current $I_{ext}$ of the beam.

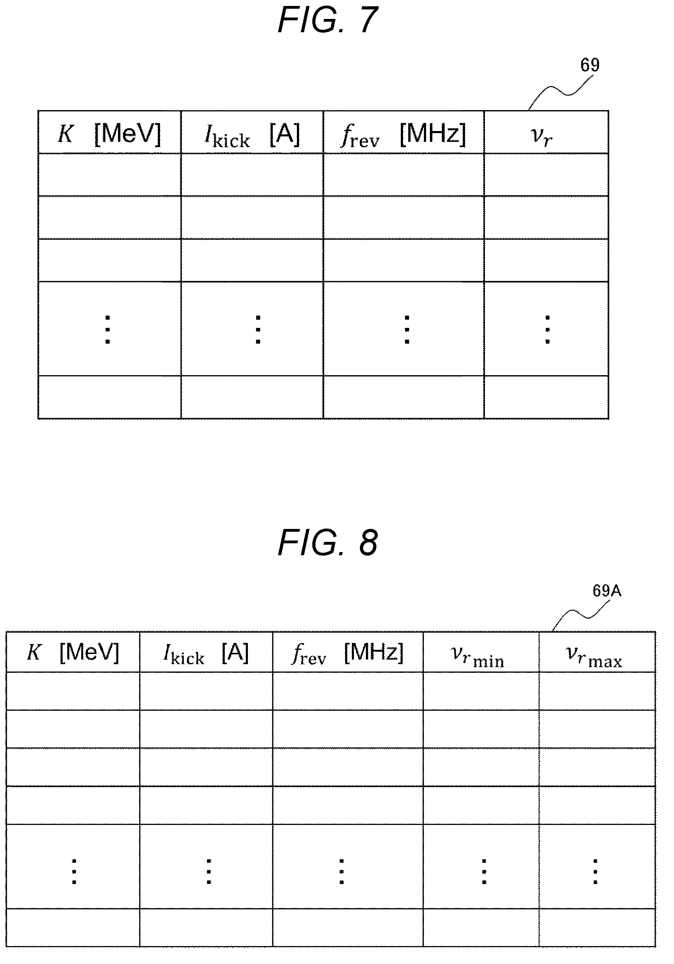

FIG. 7 illustrates an example of table data 69 used for parameter reference according to the present embodiment.

In the present embodiment, a frequency of the radio frequency disturbance electric field can be determined based on a parameter referred to from the table data 69 based on the previously obtained circulation frequency or kinetic energy. More specifically, in the table data 69 of FIG. 7, for example, center energy K of the beam, the kicker coil current $I_{kicker}$ required to reach the radio-frequency kicker 83, the circulation frequency $f_{rev}$ of the beam, and the horizontal tune $v_r$ of the beam are stored in association with each other in the radio frequency extraction voltage control device 24.

As a result, the center energy K of the beam to be applied is used as an index with respect to the obtained kinetic energy of the beam, but the circulation frequency $f_{rev}$ of the beam may also be used.

In addition, in a case where the horizontal tune $v_r$ varies depending on a multipolar field at the time of application to the radio-frequency kicker 83, as illustrated in FIG. 8, a minimum value $v_{rmin}$ and a maximum value $v_{rmax}$ are set as table data 69A, and band noise based on the minimum value $v_{rmin}$ and the maximum value $v_{rmax}$ is generated, for example, so that more efficient beam extraction is expected.

Furthermore, a radio-frequency voltage for extraction having an arbitrary power spectrum may be applied not only through the band noise but also through a function generator or the like. At this time, parameters required for expressing each spectrum are stored as the table data 69.

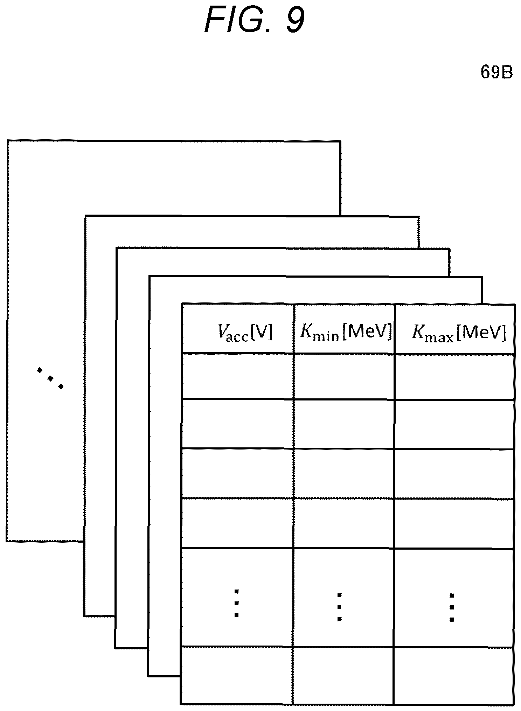

In the accelerator according to the present embodiment, the beam is synchrotron-oscillated in a radio frequency bucket, so that the beam has a certain width with respect to the center energy of the beam. The width depends on a width of the radio frequency bucket in an energy direction, and is changed by a radio frequency acceleration voltage $V_{acc}$. Therefore, by storing an upper limit and a lower limit of the kinetic energy of the beam with respect to the radio frequency acceleration voltage $V_{acc}$ for each center energy as table data 69B as illustrated in FIG. 9, the kicker coil current and the frequency $f_{ext}$ of the radio-frequency voltage for extraction covering the energies is determined based on the pieces of table data 69 and 69A illustrated in FIGS. 7 and **8,** so that the beam extraction efficiency is further improved.

Next, effects of the present embodiment will be described.

The circular accelerator 30 according to the first embodiment of the present invention described above is a device that applies a static magnetic field that circulates a charged particle beam, a radio frequency acceleration electric field that is frequency-modulated and accelerates the charged particle beam, and a radio frequency disturbance electric field that extracts the charged particle beam, in which a circulation frequency of the charged particle beam circulating inside the circular accelerator 30 or kinetic energy of the charged particle beam is obtained after stopping the radio frequency acceleration electric field, and the radio frequency disturbance electric field is generated according to the obtained circulation frequency or kinetic energy.

Hitherto, the radio frequency disturbance electric field has a ratio of 1:1 with respect to the radio frequency acceleration electric field, but in the present invention, the circulation frequency or kinetic energy of the circulating charged particle beam after acceleration is obtained, and the radio frequency disturbance electric field is generated according to the obtained circulation frequency or kinetic energy. Therefore, even in a case where there is a frequency shift, the betatron oscillation amplitude can be increased by a more appropriate radio frequency disturbance electric field. Therefore, final beam utilization efficiency can be improved.

In addition, since the circular accelerator further applies the dynamic magnetic field that kicks the charged particle beam circulating in the circular accelerator 30 to the generation region of the radio frequency disturbance electric field, it is possible to achieve extraction of the charged particle beam with high accuracy.

Furthermore, by obtaining the circulation frequency or the kinetic energy by measuring the circulation frequency or the kinetic energy, obtaining the circulation frequency or the kinetic energy based on the command value to the dee electrode 12 and the dummy dee electrode 13 to which the radio frequency acceleration electric field is applied, obtaining the circulation frequency or the kinetic energy based on the measured value of the radio frequency acceleration electric field, or obtaining the circulation frequency or the kinetic energy based on the stop time of the radio frequency acceleration electric field, it is possible to apply an accurate radio frequency acceleration voltage suitable for extracting a more circulating charged particle beam.

In addition, by determining the frequency of the radio frequency disturbance electric field based on the parameter referred to from the table data 69 based on the rotation frequency or the kinetic energy, rapid and accurate charged particle beam extraction can be achieved.

<Second Embodiment>

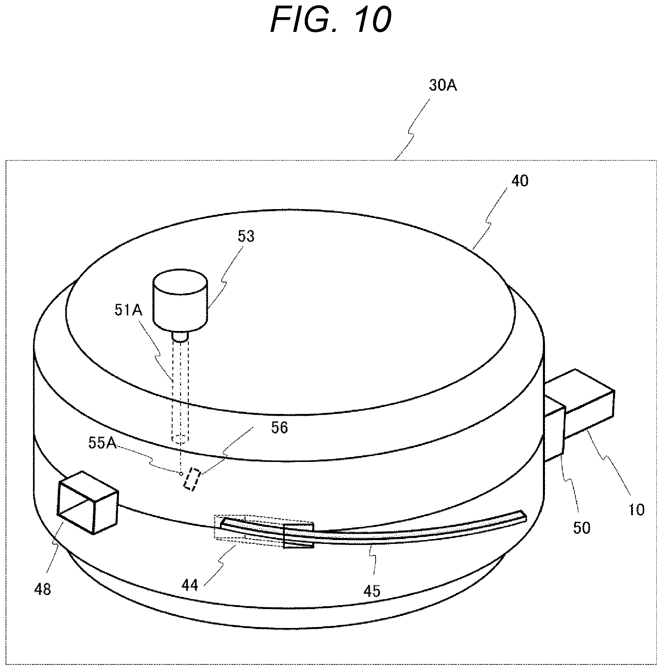

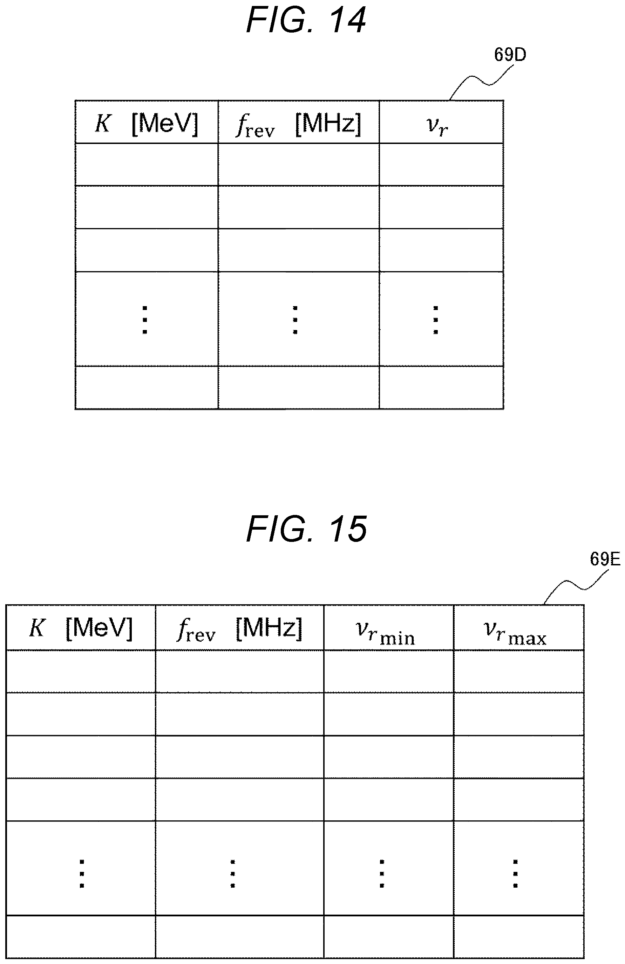

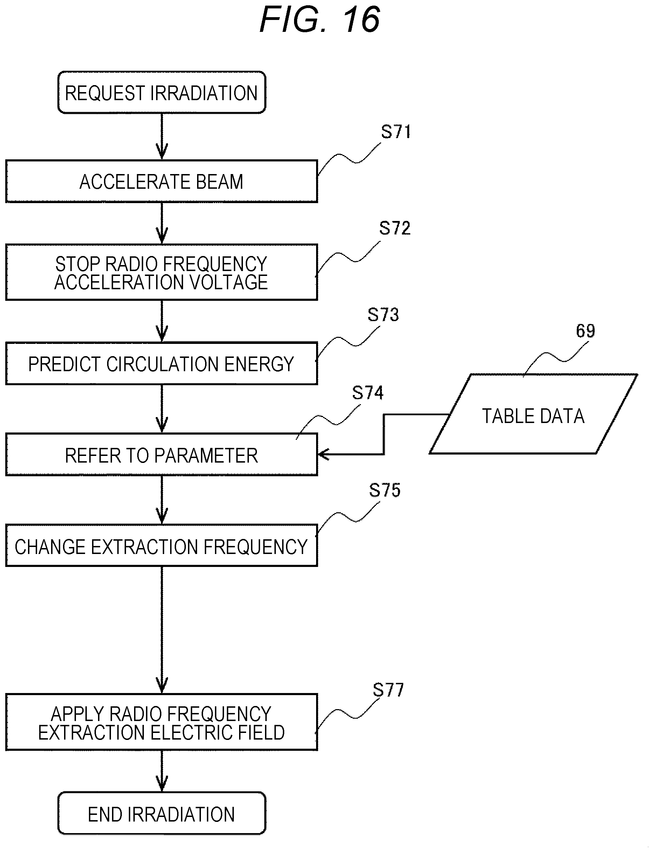

An accelerator according to a second embodiment of the present invention will be described with reference to FIGS. 10 to 16. In the present embodiment, a description of the same configuration as that of the first embodiment will be omitted, and only different configurations will be described.

A circular accelerator 30A according to the present embodiment illustrated in FIG. 10 is an eccentric orbit type accelerator in which a circulation orbit of a charged particle beam is eccentric in one direction from the center of the circular accelerator 30A, and a main magnetic field is formed so as to be eccentric toward a beam extraction path entrance 82.

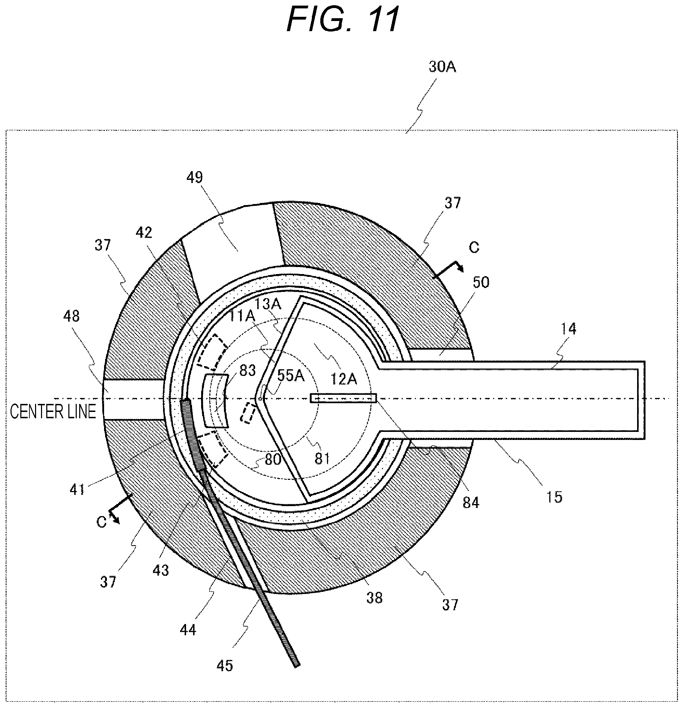

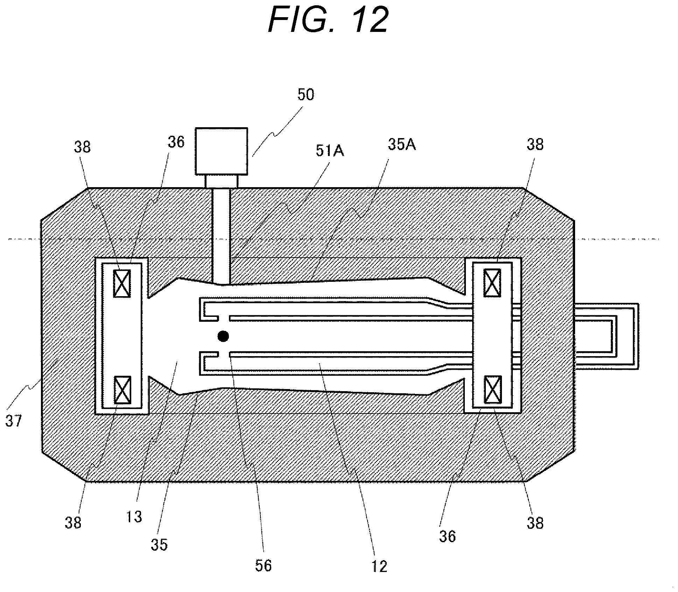

FIGS. 11 and 12 illustrate a cross-sectional configuration of the eccentric orbit type accelerator. Structural changes from FIG. 2 include shapes of a dee electrode 12A and a dummy dee electrode 13A, and a shape of an acceleration gap 11A formed therebetween. Here, a line passing through the center of a circle of an acceleration region is defined as a center line. An ion injection portion 55A and a low energy beam transport system 51A are disposed closer to the beam extraction path entrance 82 than the center of the acceleration region on the center line.

Although not illustrated for convenience of illustration, shapes of vertically opposing surfaces of a main magnetic pole 35A for forming a magnetic field described below is also greatly different from that of the first embodiment.

The acceleration gap 11A formed between the dee electrode 12A and the dummy dee electrode 13A facing each other is installed along a synchronous phase line. More specifically, the dee electrode 12A has a hollow shape such as a fan shape with a tip near the center of a concentric orbit and a radius along the synchronous phase line. In addition, the dummy dee electrode 13A has a shape facing the dee electrode 12A.

In a region where energy of the beam is low, the beam is close to the concentric orbit centered around the vicinity of the ion injection portion 55A similarly to a cyclotron, but orbits of larger energy are densely aggregated in the vicinity of the beam extraction path entrance 82, and conversely, the orbits of the respective energies are separated from each other in the vicinity of an inner conductor 14. A point where the orbits are densely aggregated is referred to as an aggregation region, and a region where the orbits are discrete is referred to as a discrete region. By adopting such orbit arrangement and extracting the beam from the vicinity of the aggregation region, a required beam kicking amount can be reduced, so that it is possible to easily perform beam extraction with variable energy.

In order to generate the above-described orbit configuration and stable vibration around the orbit, in the accelerator according to the present embodiment, distribution in which the main magnetic field decreases toward a radially outer circumferential side is formed by a shape of the main magnetic pole 35 and a trim coil or magnetic pole piece installed on a surface thereof. In addition, the main magnetic field has a constant value on a line along a design orbit. Therefore, the design orbit is circular.

Next, a beam extraction method will be described. For beam extraction, a radio-frequency kicker 83 installed near the aggregation region where all beam orbits of extraction energies are aggregated, a peeler magnetic field region 42 and a regenerator magnetic field region 43 disposed on both sides thereof, a septum coil 41, and a high energy beam transport system 45 are used.

In the present embodiment, a configuration of the radio-frequency kicker 83 among the above elements used for extraction is different from that of the first embodiment. Further, a kicker coil 85 does not have to be provided. A beam extraction procedure is basically substantially the same as that described in the first embodiment, but if a timing of cutting off an acceleration radio frequency voltage and a timing of start of application of a radio frequency voltage to the radio-frequency kicker 83 are shifted forward, a beam of arbitrary energy can be extracted. When the application of the radio frequency voltage is started, an amplitude of betatron oscillation of a beam of desired energy is increased by the radio-frequency kicker 83. Eventually, the beam reaches the peeler magnetic field region 42 and the regenerator magnetic field region 43 and is extracted.

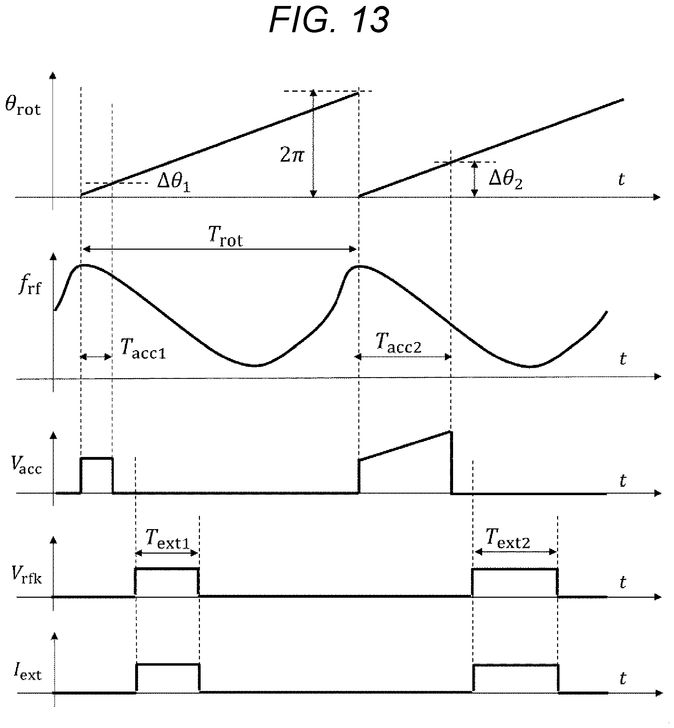

FIGS. 13 to 16 illustrate a case where the kicker coil 85 is not used according to the present embodiment in FIGS. 5 to 8. In a case where the kicker coil 85 is applied to the present embodiment, the same control and table data as those in FIGS. 5 to 8 are used.

Other configurations and operations are substantially the same as those of the accelerator according to the first embodiment described above, and details thereof are omitted.

Also in the accelerator according to the second embodiment of the present invention, substantially the same effects as those of the accelerator according to the first embodiment described above can be obtained.

In addition, since the circulation orbit of the charged particle beam is eccentric in a certain direction from the center of the circular accelerator 30A, the circulation orbits of the beams are aggregated in the aggregation region, and thus, it is possible to bend the charged particle beam to an extraction orbit with a smaller local magnetic field than an unaggregated orbit, and the extraction becomes very easy. In particular, in the aggregation region, an interval between closed orbits of the beams is narrower than that in the related art, an ion beam having predetermined energy can be stably and easily extracted even when the energy of the ion beam is over a wide range.

<Third Embodiment>

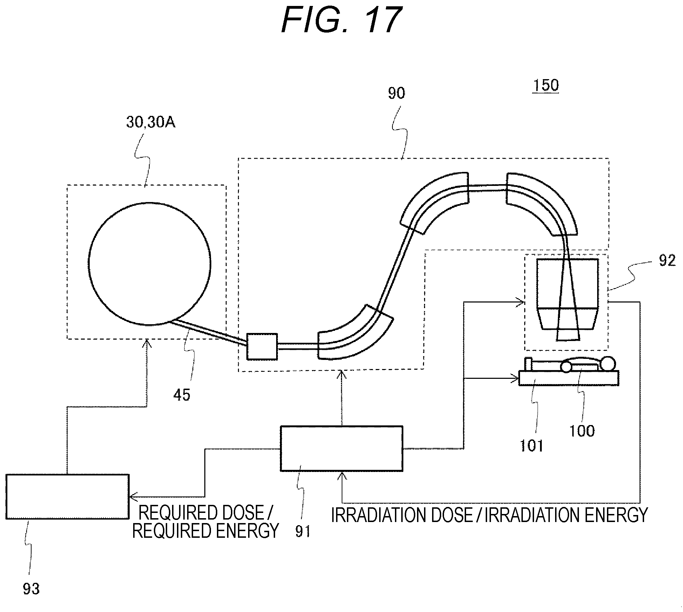

A particle therapy apparatus according to a third embodiment of the present invention will be described with reference to FIG. 17. FIG. 17 is an overall configuration diagram of the particle therapy apparatus according to the present embodiment.

A particle therapy apparatus 150 according to the present embodiment illustrated in FIG. 17 includes the circular accelerator 30 according to the first embodiment or the circular accelerator 30A according to the second embodiment, a rotating gantry 90, an irradiation device 92 including a scanning magnet, a treatment couch 101, and a control device 91 that controls these components.

In the particle therapy apparatus 150, a beam extracted from the circular accelerator 30 or 30A is transported to the irradiation device 92 by the rotating gantry 90. The transported ion beam is formed in accordance with a target volume by adjustment of the irradiation device 92 and energy of the beam, and the target volume of a patient 100 lying on the treatment couch 101 is irradiated with a predetermined amount of beam.

The irradiation device 92 includes a dose monitor and monitors a dose irradiated for each irradiation spot on the patient 100. The control device 91 calculates a required dose for each irradiation spot based on dose data and uses the calculated dose as input data to an accelerator control device 93. The accelerator control device 93 controls injection, acceleration, and extraction of a charged particle beam in the circular accelerator 30 or 30A, and supplies a beam of a required dose and energy.

The configuration and operation of the circular accelerator 30 or 30A are substantially the same as those of the first embodiment or the second embodiment described above, and details thereof will be omitted.

<Others>

Note that the present invention is not limited to the embodiments described above, but includes various modified examples. For example, the embodiments described above have been described in detail in order to explain the present invention in an easy-to-understand manner, and the present invention is not necessarily limited to those having all the configurations described.

In addition, a part of a configuration of an embodiment can be replaced with a configuration of another embodiment, and a configuration of an embodiment can be added with a configuration of another embodiment. Further, a part of the configuration of each embodiment can be added with another configuration, can be deleted, and can be replaced with another configuration.

The embodiments of the present invention may have the following aspects.

(1) A circular accelerator that applies a static magnetic field that circulates a charged particle beam, a radio frequency acceleration electric field that is frequency-modulated and accelerates the charged particle beam, and a radio frequency disturbance electric field that extracts the charged particle beam, in which a circulation frequency of the charged particle beam circulating inside the circular accelerator or kinetic energy of the charged particle beam is obtained after stopping the radio frequency acceleration electric field, and the radio frequency disturbance electric field is generated according to the obtained circulation frequency or kinetic energy.

(2) The circular accelerator according to (1), in which the circular accelerator further applies a dynamic magnetic field that kicks the charged particle beam circulating in the circular accelerator to a generation region of the radio frequency disturbance electric field.

(3) The circular accelerator according to (1) or (2), in which the circulation frequency or the kinetic energy is obtained by measuring the circulation frequency or the kinetic energy.

(4) The circular accelerator according to any one of (1) to (3), in which the circulation frequency or the kinetic energy is obtained based on a command value for an acceleration electrode to which the radio frequency acceleration electric field is applied.

(5) The circular accelerator according to any one of (1) to (4), in which the circulation frequency or the kinetic energy is obtained based on a measured value of the radio frequency acceleration electric field.

(6) The circular accelerator according to any one of (1) to (5), in which the circulation frequency or the kinetic energy is obtained based on a stop time of the radio frequency acceleration electric field.

(7) The circular accelerator according to any one of (1) to (6), in which a frequency of the radio frequency disturbance electric field is determined based on a parameter referred to from table data based on the circulation frequency or the kinetic energy.

(8) The circular accelerator according to any one of (1) to (7), in which a circulation orbit of the charged particle beam is eccentric in one direction from a center of the circular accelerator.

(9) A particle therapy apparatus including: the circular accelerator according to any one of (1) to (8).

Reference Signs List

10 radio frequency acceleration cavity
11, 11A acceleration gap
12, 12A dee electrode (acceleration electrode)
13, 13A dummy dee electrode (acceleration electrode)
14 inner conductor
15 outer conductor
20 radio frequency acceleration voltage control device
21 radio frequency acceleration power supply
22 radio frequency acceleration power supply control device
23 radio frequency acceleration voltage frequency control device
24 radio frequency extraction voltage control device
25 radio frequency extraction power supply
26 radio frequency extraction power supply control device
27 radio frequency extraction voltage frequency control device
30, 30A circular accelerator
35, 35A main magnetic pole
36 cryostat
37 yoke
38 main coil
40 main magnet
41 septum coil
42 peeler magnetic field region
43 regenerator magnetic field region
44 beam through hole
45 high energy beam transport system
46 resonance suppression magnetic field region
48 coil through hole

49 vacuum evacuation through hole
50 radio frequency system through hole
51, 51A low energy beam transport system
53 ion source
55, 55A ion injection portion
56 electrostatic inflector
60 variable capacitor
61 variable capacitor control device
69, 69A, 69B, 69C, 69D table data
80 minimum energy orbit
81 maximum energy orbit
82 beam extraction path entrance
83 radio-frequency kicker
84 measurement electrode
85 kicker coil
90 rotating gantry
91 control device
92 irradiation device
93 accelerator control device
100 patient
101 treatment couch
150 particle therapy apparatus

**Claims**

1. A circular accelerator that applies a static magnetic field that circulates a charged particle beam, a radio frequency acceleration electric field that is frequency-modulated and accelerates the charged particle beam, and a radio frequency disturbance electric field that extracts the charged particle beam,

   wherein a circulation frequency of the charged particle beam circulating inside the circular accelerator or kinetic energy of the charged particle beam is obtained after stopping the radio frequency acceleration electric field, and
   the radio frequency disturbance electric field is generated according to the obtained circulation frequency or kinetic energy.

2. The circular accelerator according to claim 1, wherein the circular accelerator further applies a dynamic magnetic field that kicks the charged particle beam circulating in the circular accelerator to a generation region of the radio frequency disturbance electric field.

3. The circular accelerator according to claim 1, wherein the circulation frequency or the kinetic energy is obtained by measuring the circulation frequency or the kinetic energy.

4. The circular accelerator according to claim 1, wherein the circulation frequency or the kinetic energy is obtained based on a command value for an acceleration electrode to which the radio frequency acceleration electric field is applied.

5. The circular accelerator according to claim 1, wherein the circulation frequency or the kinetic energy is obtained based on a measured value of the radio frequency acceleration electric field.

6. The circular accelerator according to claim 1, wherein the circulation frequency or the kinetic energy is obtained based on a stop time of the radio frequency acceleration electric field.

7. The circular accelerator according to claim 1, wherein a frequency of the radio frequency disturbance electric field is determined based on a parameter referred to from table data based on the circulation frequency or the kinetic energy.

8. The circular accelerator according to claim 1, wherein a circulation orbit of the charged particle beam is eccentric in one direction from a center of the circular accelerator.

9. A particle therapy apparatus comprising the circular accelerator according to any one of claims 1 to 8.

# FIG. 1

# FIG. 2

# FIG. 3

# FIG. 4

# FIG. 5

REQUEST IRRADIATION

ACCELERATE BEAM — S71

STOP RADIO FREQUENCY ACCELERATION VOLTAGE — S72

PREDICT CIRCULATION ENERGY — S73

REFER TO PARAMETER — S74

TABLE DATA — 69

CHANGE EXTRACTION FREQUENCY — S75

APPLY KICKER COIL CURRENT — S76

APPLY RADIO FREQUENCY EXTRACTION ELECTRIC FIELD — S77

END IRRADIATION

## FIG. 6

## FIG. 7

69

| $K$ [MeV] | $I_{\text{kick}}$ [A] | $f_{\text{rev}}$ [MHz] | $\nu_r$ |
|---|---|---|---|
|  |  |  |  |
|  |  |  |  |
|  |  |  |  |
| $\vdots$ | $\vdots$ | $\vdots$ | $\vdots$ |
|  |  |  |  |

## FIG. 8

69A

| $K$ [MeV] | $I_{\text{kick}}$ [A] | $f_{\text{rev}}$ [MHz] | $\nu_{r\,\text{min}}$ | $\nu_{r\,\text{max}}$ |
|---|---|---|---|---|
|  |  |  |  |  |
|  |  |  |  |  |
|  |  |  |  |  |
|  |  |  |  |  |
| $\vdots$ | $\vdots$ | $\vdots$ | $\vdots$ | $\vdots$ |
|  |  |  |  |  |

# FIG. 9

69B

| $V_{acc}$[V] | $K_{min}$[MeV] | $K_{max}$[MeV] |
|---|---|---|
|  |  |  |
|  |  |  |
|  |  |  |
|  |  |  |
| ⋮ | ⋮ | ⋮ |
|  |  |  |

# FIG. 10

# FIG. 11

# FIG. 12

# *FIG. 13*

# FIG. 14

69D

| $K$ [MeV] | $f_{\mathrm{rev}}$ [MHz] | $v_r$ |
|---|---|---|
| | | |
| | | |
| | | |
| $\vdots$ | $\vdots$ | $\vdots$ |
| | | |

# FIG. 15

69E

| $K$ [MeV] | $f_{\mathrm{rev}}$ [MHz] | $v_{r\,\mathrm{min}}$ | $v_{r\,\mathrm{max}}$ |
|---|---|---|---|
| | | | |
| | | | |
| | | | |
| | | | |
| $\vdots$ | $\vdots$ | $\vdots$ | $\vdots$ |
| | | | |

# *FIG. 16*

REQUEST IRRADIATION

S71

ACCELERATE BEAM

S72

STOP RADIO FREQUENCY
ACCELERATION VOLTAGE

S73

PREDICT CIRCULATION ENERGY

S74

REFER TO PARAMETER

69

TABLE DATA

S75

CHANGE EXTRACTION FREQUENCY

S77

APPLY RADIO FREQUENCY
EXTRACTION ELECTRIC FIELD

END IRRADIATION

# FIG. 17

REQUIRED DOSE / REQUIRED ENERGY

IRRADIATION DOSE / IRRADIATION ENERGY

## INTERNATIONAL SEARCH REPORT

| International application No. |
|---|
| **PCT/JP2023/000454** |

**A. CLASSIFICATION OF SUBJECT MATTER**

*H05H 13/02*(2006.01)i; *A61N 5/10*(2006.01)i
FI: H05H13/02; A61N5/10 H

According to International Patent Classification (IPC) or to both national classification and IPC

**B. FIELDS SEARCHED**

Minimum documentation searched (classification system followed by classification symbols)

H05H13/02; H05H13/00; H05H7/10; A61N5/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2020-202015 A (HITACHI, LTD.) 17 December 2020 (2020-12-17)<br>entire text, all drawings | 1-9 |
| A | JP 2020-38797 A (HITACHI, LTD.) 12 March 2020 (2020-03-12)<br>entire text, all drawings | 1-9 |
| A | JP 2022-67810 A (HITACHI, LTD.) 09 May 2022 (2022-05-09)<br>paragraphs [0014]-[0080], fig. 1-9 | 1-9 |
| A | JP 2019-133745 A (HITACHI, LTD.) 08 August 2019 (2019-08-08)<br>paragraphs [0014]-[0069], fig. 1-13 | 1-9 |
| A | JP 2022-26175 A (HITACHI, LTD.) 10 February 2022 (2022-02-10)<br>paragraphs [0018]-[0111], fig. 1-15 | 1-9 |
| A | JP 2018-85326 A (ION BEAM APPLICATIONS S. A.) 31 May 2018 (2018-05-31)<br>abstract, fig. 1 | 1-9 |

☐ Further documents are listed in the continuation of Box C.    ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **16 February 2023** | **28 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/000454**

| Patent document cited in search report | | | Publication date (day/month/year) | Patent family member(s) | | | Publication date (day/month/year) |
|---|---|---|---|---|---|---|---|
| JP | 2020-202015 | A | 17 December 2020 | (Family: none) | | | |
| JP | 2020-38797 | A | 12 March 2020 | US<br>entire text, all drawings<br>WO | 2021/0196984<br><br>2020/049755 | A1<br><br>A1 | |
| JP | 2022-67810 | A | 09 May 2022 | WO<br>paragraphs [0014]-[0080], fig. 1-9 | 2022/085273 | A1 | |
| JP | 2019-133745 | A | 08 August 2019 | US<br>paragraphs [0026]-[0082], fig. 1-13<br>WO<br>CN | 2021/0195725<br><br><br>2019/146211<br>111279801 | A1<br><br><br>A1<br>A | |
| JP | 2022-26175 | A | 10 February 2022 | (Family: none) | | | |
| JP | 2018-85326 | A | 31 May 2018 | US<br>abstract, fig. 1<br>EP<br>CN<br>CN | 2018/0098413<br><br>3307031<br>107920412<br>208927452 | A1<br><br>A1<br>A<br>U | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11160159 B

- JP 2019133745 A

**Non-patent literature cited in the description**

- **W. KLEEVEN**. The IBA Superconducting Synchro-cyclotron Project S2C2. *Proceedings of Cyclotrons*, 2013